# EUROPEAN PATENT SPECIFICATION

(11) **EP 3 266 762 B1**
(45) Date of publication and mention of the grant of the patent: **22.05.2019**
(21) Application number: 17179322.7
(22) Date of filing: 03.07.2017
(51) Int. Cl.: C07C 233/91, C11D 9/44, C11D 17/06

(54) **ACTIVATORS FOR PEROXYGENATED COMPOUNDS**
AKTIVATOREN FÜR PERSAUERSTOFFVERBINDUNGEN
ACTIVATEURS POUR COMPOSÉS PEROXYGÉNÉS

(30) Priority: 06.07.2016 IT 201600070454
(43) Date of publication of application: 10.01.2018
(73) Proprietor: 3V SIGMA S.p.A, 20121 Milano (IT)
(72) Inventor: CRIVELLO, Antonella, 24121 Bergamo (IT); LANZENI, Valentina, 24121 Bergamo (IT); MAESTRI, Francesco, 24121 Bergamo (IT)
(74) Representative: Bianchetti Bracco Minoja S.r.l.

(56) References cited:
- EP-A1- 0 070 432
- WO-A1-98/16496
- WALDEMAR ADAM ET AL: "Chemiluminescence of the Labile 1,2-Dioxetanes and Epoxides Produced in the Oxidation of N-Acetylated Dihydro- and Tetrahydropyrazines by Singlet Oxygen, Dimethyldioxirane, and m-Chloroperoxybenzoic Acid", JOURNAL OF THE AMERICAN CHEMICAL SOCIETY, vol. 117, no. 38, 1 September 1995 (1995-09-01), pages 9690-9692, XP055337778, US ISSN: 0002-7863, DOI: 10.1021/ja00143a011

## Description

The present invention relates to novel activators for peroxygenated compounds and compositions containing them. The activators according to the invention, which can be used in additives and bleaching compositions in liquid or solid form, are effective on fabrics with a wide range of hydrophilic and lipophilic dirt and stains, at temperatures lower than 60°C.

### PRIOR ART

It is well known that peroxygen agents that release hydrogen peroxide in aqueous solution, such as sodium perborate, sodium percarbonate, sodium perphosphate, sodium persilicate, urea peroxide and the like, are very useful for removing stains from white and coloured fabrics. However, said bleaching agents are only very efficient at high washing temperatures, exceeding 80°C.

The whitening activity of peroxygenated compounds can be improved and made effective at temperatures lower than 60°C with the use of peroxyacid precursors called bleaching activators. An activator is typically an organic substance which, in alkaline solution, reacts with the hydroperoxide ion generated by hydrogen peroxide during washing to form a peroxyacid. The peroxyacid formed in situ then oxidises the stains on the substrate.

Numerous activators for peroxygenated compounds are described in the literature, the majority of which contain perhydrolysable acyl groups having oxybenzene sulphonated leaving groups.

The first examples of activators reported in the literature relate to some classes of esters such as the benzoyl ester of sodium phenol sulphonate (GB 836988) and sodium p-acetoxybenzene sulphonate (GB 864798). GB 855735 and GB 907356 relate to the large class of imides, which includes N,N,N',N'-tetraacetyl ethylene diamine (TAED), one of the most commonly used activators. DE 2301235 and DE 2301437 also provide examples of peracylated polyamides characterised by high stability and compatibility with optical brighteners.

Various types of activators were subsequently described, for example in DE 1695219, US 4221675, US 5041232, US5518650 and US2001046953.

The efficacy of an activator depends on a variety of factors, such as solubility, reactivity, pKa, and above all the type of peroxyacid generated during washing, which can act differently according to the type of stain. It is known (EP 068 547) that hydrophilic peroxyacids are efficient in washing hydrophilic stains (tea, fruit juice and the like) but not hydrophobic stains (grease, grass and the like), while conversely, hydrophobic peroxyacids are efficient on hydrophobic but not hydrophilic stains. Among the hydrophobic peroxyacids, it has been found that those containing a long hydrocarbon chain with a percarboxyl group at one end are more efficient than others having a different structure.

As different types of stains are to be found in the domestic and industrial environments, which may have hydrophilic or hydrophobic components, many attempts have been made to improve washing performance by combining activators with different characteristics, as reported in patents EP 105 690 and EP 106 584.

EP 257 700 describes the use of two of the activators most commonly employed in detergents, namely TAED (N,N,N',N'-tetraacetyl ethylene diamine) and sodium nonanoyloxybenzenesulphonate (NOBS). The former, in the presence of the hydroperoxide ion in the washing solution, generates peracetic acid, which is very efficient against hydrophilic stains, while the latter generates pernonanoic acid, which is very efficient against lipophilic stains.

EP 0 070 432 A1 discloses a process for the preparation of purified TAED from mixtures containing TAED by dispersing said mixtures in a suspending agent.

WO 98/16496 A1 discloses a process for the preparation of non-cyclic imides bearing two different acyl groups, useful as bleaching activators.

Journal of American Chemical Society, 1995, 117, 9690-9692 discloses the compound N,N'-bisacetyl-N,N'-bisbenzoyl ethylene diamine. The compound is isolated in the course of chemiluminiscence study of 1,2-dioxoethanes produced by the oxidation of 1,4-diacetyl-2,3-diphenyl-1,4,5,6-tetrahydropirazinaes therefore continuing with the aim of finding a more efficient activator than those currently present, which performs satisfactorily on both hydrophilic and lipophilic stains and dirt, and is safe and stable over time, maintaining its efficacy.

### DESCRIPTION OF THE INVENTION

The activators for peroxygenated compounds according to the invention are functionalised polyamines which have the advantage of generating in the washing environment, in the presence of the hydroperoxide ion, peroxyacid species able to oxidise hydrophilic and hydrophobic stains, exhibiting superior efficacy and performance to the activators commonly used in detergent and bleaching compositions.

The activators according to the invention have the following formula (I) 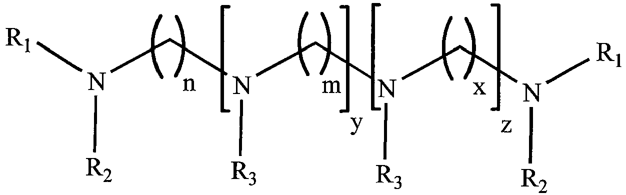
wherein R₁ is a straight or branched, saturated or unsaturated acyl group having 2 to 5 carbon atoms. This group represents the hydrophilic part of the activator.
R₂ is a straight or branched, saturated or unsaturated acyl group having 6 to 18 carbon atoms. This group represents the hydrophobic part of the activator.
R₃ is a straight or branched, saturated or unsaturated acyl or alkyl group having 2 to 18 carbon atoms;
y has a value from 0 to 3;
m, n and x independently have a value from 2 to 3;
z has a value of zero or 1.

The groups reported above can optionally be substituted with functional groups compatible with the washing environment.

The preferred compounds of formula I are those wherein z is 1. Said compounds can be represented by formula Ia

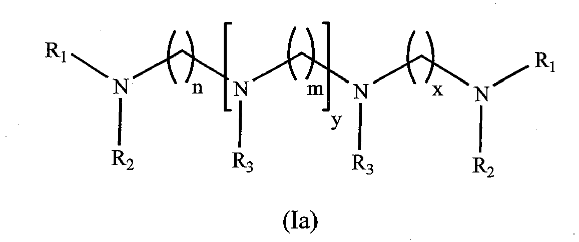

It has been analytically proved that in the bleaching activators characterised by formula (I), in the presence of the hydroperoxide ion in the washing water, perhydrolysis takes place on both carbonyl groups, i.e. hydrophilic (R₁) and hydrophobic (R₂).

R₁ preferably represents an acetyl, propionyl, 2-methyl-propionyl or butyryl group, more preferably acetyl.

R₂ is preferably a straight saturated group such as heptanoyl, octanoyl, nonanoyl, decanoyl, undecanoyl, dodecanoyl or octadecanoyl, preferably a C₈-C₁₂ acyl group, and more preferably nonanoyl.

R₃ is preferably the same as R₁ or R₂. The situation is even more advantageous if R₃ is the same as R₁. Preferably, R₁ and R₃ are acetyl.

y is preferably equal to 1 and n is equal to x. In a particularly advantageous case, n and x are equal to 3 and m is equal to 2. A particularly preferred activator of formula (Ia) has the following structure:

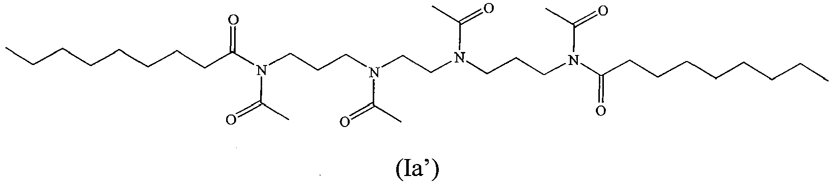

Other preferred compounds of formula I are those wherein y and z are 0, of formula (Ib).

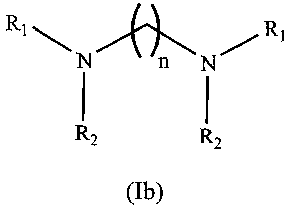

In the compounds of formula (Ib), n is preferably equal to 2. A preferred activator of formula (Ib) has the following structure:

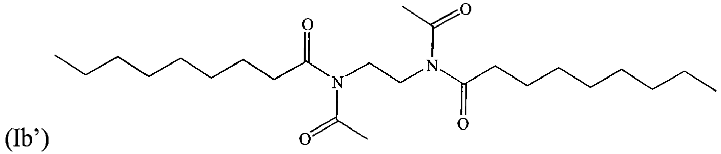

The compounds of formula (I) can be obtained by two different processes. According to a first method, the terminal amines of a polyamine of formula (II), 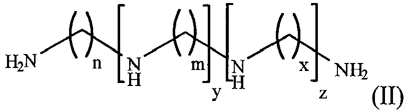 wherein n, m, x, y and z can have the values reported above, are reacted with an acylating agent such as a carboxylic acid or an ether of formula (III) 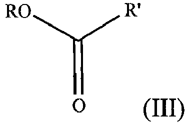 wherein R can be a hydrogen atom or an alkyl radical, R'CO can be the same as R₁ or R₂, and is preferably the same as R₂. The bisamidoamine of formula (IV) is obtained by this method.

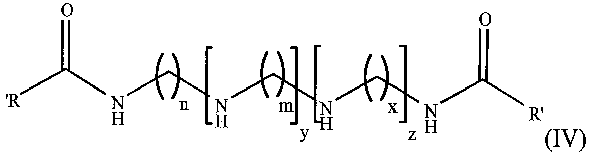

This stage of the reaction proceeds at high temperature, between 100° and 250°C, according to the reactivity of the acid or the ester, with continuous removal of the alcohol or condensed water by distillation. The process can be effected with or without a catalyst, such as phosphoric acid, at atmospheric pressure, under vacuum or under pressure. The reaction can also be effected in the presence or absence of an aprotic solvent, preferably without a solvent.

The secondary amines of the bisamidoamine thus obtained can then be reacted with anhydrides such as acetic anhydride, succinic anhydride and maleic anhydride, or with an alkylating agent such as a compound having a reactive chloro group, for example chloroacetic acid, an ester thereof or a salt thereof, a compound that can give the Michael addition, such as methyl acrylate or sodium vinyl sulphonate, or electrophiles such as propane sultone or terminal epoxy compounds such as butyl glycidyl ether or 2-ethylhexyl glycidyl ether.

The bisamidoamine, functionalised or non-functionalised on the secondary amines, can be subsequently treated with an acyl chloride (as reported in US6028192 and US7425527, for example) of formula: 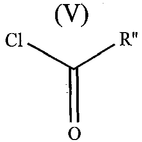 wherein R"CO is the same as R₁ if R'CO is the same as R₂. However, if R'CO is the same as R₁, R"CO is the same as R₂. Alternatively, the bisamidoamine can be treated with an anhydride of structure 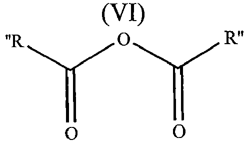 wherein R"CO can be R₁ or R₂ as reported above. If the process starts with bisamidoamine with the free secondary amines, R₃ is the same as R"CO.

The second process which can be used to obtain the compounds of formula (I)) requires R₃ to be the same as R₁ or R₂. According to this method, the polyamine of formula (II) is first treated with an anhydride of structure (VI) or an acyl chloride of structure (V) to functionalise all the amines present. The imide can then be obtained by the same method as reported above, again using an anhydride or an acyl chloride having a different R''' group, which must be different from R".

Conversely, the compounds of formula (Ib) can be obtained as described for those with structure (Ia), except for the possibility of functionalising the secondary amino groups.

Depending on the substituents, the activator can be in liquid, solid or resin form.

If the product is not solid, such as the product of formula (Ia'), it can be formulated in liquid compositions or alternatively adsorbed on water-insoluble inorganic compounds containing silicone-oxygen bonds, as described in US 4207199. The inorganic compound can be amorphous or crystalline, provided that it has a sufficiently large area to adsorb the product in quantities exceeding 25%. The adsorbent substance must also be substantially anhydrous to prevent breakdown of the activator by hydrolysis.

The inorganic compounds which can be used for this purpose are aluminosilicates, sodium aluminosilicates, calcium aluminosilicates and magnesium aluminosilicates such as zeolite A and zeolite X, preferably zeolite A.

If the activator is solid, such as the product of formula (Ib'), to protect it against humidity and other factors which could cause its deterioration, it can be coated with a substance that is soluble in the washing liquid or at least swells therein (GB 907358). Substances suitable for coating the activator, some of which are normally part of the washing composition, include polyvinyl alcohol, carboxymethylcellulose, cetyl alcohol, stearic acid, palmitic acid and water-soluble polyethylene glycols (e.g. Carbowax® 4000 and Carbowax® 6000).

The invention also relates to bleaching compositions comprising an activator of formula (I) and other conventional ingredients.

The polyamines according to the invention can be used in combination with hydrogen peroxide or inorganic peroxygenated compounds such as perborates, percarbonates, persilicates, perphosphates, persulphates and peroxides of alkaline metals, as oxidising agents and bleaches in the various fields in which peroxygenated compounds can be used.

Specifically, the consumer products which can benefit from the invention include products for washing and bleaching fabrics of all kinds, whether natural or synthetic, all household surfaces including sinks, floors, toilet seats, toilets and cookers, and kitchenware such as glasses, cutlery, plates and the like. The bleaching system according to the invention can also be applied for industrial use in the paper pulp bleaching process. The system according to the invention can be included in various forms and products such as powdered detergents, sheets or other substrates, bags, tablets, anhydrous liquids and non-ionic surfactants. The system according to the invention can be combined with other bleaching activators or catalysts, such as TEAD and NOBS.

The polyamides are preferably used for washing and bleaching natural and synthetic fabrics.

The compounds of formula (I) can be formulated in bleaching compositions in combination with ingredients normally used in said compositions as a source of hydrogen peroxide, conventional bleaching activators, surfactants, builders, structuring agents and other additives.

Examples of peroxide sources include sodium perborate, preferably mono- or tetrahydrate, sodium carbonate peroxyhydrate or the equivalent percarbonate salt, sodium pyrophosphate peroxyhydrate, urea peroxyhydrate, sodium peroxide and hydrogen peroxide. A mixture of some of said additives can also be used. Sodium perborate tetrahydrate and sodium perborate monohydrate are particularly suitable for these purposes. Sodium perborate monohydrate is preferable, because it has excellent stability and rapid solubility in water.

The concentration of the hydrogen peroxide source can range from 1% to 90% by weight of the bleaching composition. The hydrogen peroxide source and the activator are typically present in a ratio ranging from 1500:1 to 1:20, expressed as the molar ratio between the hydrogen peroxide released by the peroxygenated compound and the activator. Said ratio is preferably between 150:1 and 1:1.

The bleaching activators can be combined with one or more bleaching activators commonly used for said purposes. Examples of activators include N-acetylated and O-acetylated compounds such as multiacetylated alkylenediamines like N,N,N',N'-tetraacetyl ethylene diamine (TAED), acetylated hydantoin derivatives, diketopiperazine, carboxylic acid anhydrides, carboxylic acid esters such as sodium nonanoyloxybenzenesulphonate (NOBS) and the compounds described in US 4483778, some classes of esters such as the benzoyl ester of sodium phenol sulphonate (GB 836988) and sodium *p*-acetoxybenzene sulphonate (GB 864798).

Other bleaching catalysts are iminium salts, in particular 3,4-dihydroxyquinoline salts, described in US 5482515 and US 5576282, or manganese-based compounds. Other bleaching activators include oxaziridine salts, described in WO 01/16110, sulphonimines and sulphanimines, described in US 5041232 and WO 97/41199.

The commercially available surfactants which can be used according to the invention are described, for example, in "Surface active Agents and Detergents", volume I and II, by Schwartz, Perry and Berch.

The surfactants can be of natural or synthetic derivation, anionic, cationic, amphoteric, non-ionic or zwitterionic, or a mixture thereof. The total quantity of surfactants can be up to 80% by weight, preferably ranging from 1% to 40% by weight of the composition.

Synthetic anionic surfactants are usually water-soluble sulphated or sulphonated salts of alkaline metals, having alkyl radicals containing 8 to 22 carbon atoms. Examples of synthetic anionic detergent compounds are sodium and ammonium alkyl sulphate; sodium and ammonium alkyl (C₉-C₂₀) benzene sulphonate, especially straight sodium alkyl (C₁₀-C₁₅) benzene sulphonate; sodium alkyl glyceryl ether sulphate; sodium coco sulphate and sulphonate monoglyceride; sodium and ammonium salt of fatty alcohol sulphuric acid ester (C₉-C₁₈), especially products deriving from the reaction with ethylene oxide; the products of the reaction between fatty acids, such as fatty acid coco esters, and isethionic acid, neutralised with sodium hydroxide; sodium and ammonium salt of methyl taurine fatty acid amide; alkene monosulphonates, such as those deriving from the reaction between alpha olefins (C₈-C₂₀) and sodium bisulphite and those deriving from the reaction between paraffins and SO₃ and Cl₂ and then hydrolysed with a base to produce a sulphonate; C₇-C₁₂ dialkylsulphosuccinate sodium and ammonium salt; and olefin sulphonate. The most suitable anionic surfactants are sodium (C₁₁-C₁₅) alkylbenzenesulphonate, sodium (C₁₆-C₁₈) alkylsulphate and sodium (C₁₆-C₁₈) alkyl ether sulphate.

Examples of suitable non-ionic surfactants, preferably combined with one or more anionic surfactants, include, in particular, the products of the reaction between ethylene oxide and alkyl (C₆-C₂₂)phenol, generally 2-25 EO (2 to 25 units of ethylene oxide per molecule); the products deriving from condensation of primary or secondary straight aliphatic alcohol (C₈-C₁₈) with ethylene oxide, generally 2-30 EO, and the products deriving from condensation of ethylene oxide with the products of the reaction between propylene oxide and ethylenediamine. Other non-ionic surfactants include alkylpolyglycosides, polyhydroxy fatty acid amides, long-chain tertiary amine oxides and dialkyl sulphoxides.

Amphoteric and zwitterionic surfactants can be included in the composition according to the invention, but usually at low concentrations.

Salts of fatty acids (such as coco fatty acids) can be incorporated in the composition according to the invention from 0.5% to 30% by weight. They are particularly indicated in combination with anionic or non-ionic surfactants.

The compositions according to the invention can also contain sequestering agents. In particular, they can contain one or more organic or inorganic sequestering agents, such as sodium or potassium tripolyphosphate, sodium and potassium pyrophosphate, sodium and potassium orthophosphate, sodium carbonate, nitrilotriacetic acid sodium salt, sodium citrate, carboxymethylmalonate, carboxymethylsuccinate, tartrate mono- and disuccinates, oxydisuccinates, crystalline or amorphous aluminosilicates, and combinations thereof.

Carboxyl polymers or carboxyl copolymers can be included, with the function of structuring powders. Polyacrylic acids and acrylic-maleic polymers and alkyl metals or salts thereof are particularly preferred.

In addition to the ingredients already mentioned, the detergent composition according to the invention can contain conventional additives normally used in a detergent, in the well-known quantities. Examples of said additives, described in US 3936537, include foaming agents such as alkanolamines, antifoaming agents such as alkylphosphates or silicones, antiredeposition agents such as carboxymethylcellulose sodium salt, stabilisers such as tetraacetic ethylenediamine, conditioning agents, inorganic salts (sodium sulphate), optical brighteners, fragrances, enzymes such as protease, cellulase, lipase and amylase, germicides, colorants, pH regulators, corrosion inhibitors, film-forming agents and silicones.

The preferred compositions according to the invention contain, in addition to a compound of formula I, a peroxygenated compound able to produce hydrogen peroxide in an aqueous medium. The preferred ratio between peroxygenated compound and compound of formula (I) is between 50:1 and 1:50. The peroxygenated compounds are typically present in percentages ranging from 1% to 70% by weight, the compounds of formula (I) in percentages ranging from 0.1% to 60%, and the surfactants in percentages ranging from 0.1% to 60% by weight.

The bleaching catalysts can be present in percentages ranging from 0.001 to 30% by weight.

The invention will now be further described with reference to the following examples:

### Example 1: Synthesis of N,N'-Bis[(3-nonanoylamido)propyl]ethylenediamine

55.8 g of pelargonic acid (96%, 0.34 mol) is loaded into a 500 ml four-necked flask equipped with mechanical stirrer, thermometer, nitrogen valve and condenser connected to a Dean-Stark trap. 30.0 g of N,N'-Bis(3-aminopropyl)ethylenediamine (98%, 0.17 mol) is slowly added to the acid under stirring, initially at 100°C. The temperature is then increased to 170-175°C in the space of 20 min. When the temperature reaches about 165°C water begins to form, and is collected in the trap. The reaction mixture is maintained at 170-175°C for 5 hours. The progress of the reaction is monitored by the quantity of water collected in the trap and by gas chromatography. The pale yellow solid obtained can be purified of any cyclic products formed during the reaction by crushing in ethyl acetate, and 52.4 g is obtained (67% of the theoretical yield). Alternatively it can be used without further purification.

### Example 2: Synthesis of tetraacetylated N,N'-Bis[(3-nonanoylamido)propyliethylenediamine (Ia')

The acetic anhydride (345 g, 3.38 mol) is loaded into a 500 ml four-necked flask equipped with mechanical stirrer, thermometer, fractionating column containing 4 Sulzer elements, and compensated dropping funnel. The crude N,N'-Bis[(3-nonanoylamido)propyl]-ethylenediamine obtained as reported in example 1 (80.2 g) is dripped at 50°C, maintaining the diamide at 100°C, in the space of an hour. During this time the reaction mixture reaches 65°, and concentrated H₂SO₄ (1.6 g) is added. A vacuum is applied (residual pressure about 11999 Pa (90 mm Hg)) and the acetic acid is distilled as it forms, adding fresh acetic anhydride to maintain the same excess throughout. When the reaction is complete, the remaining acetic anhydride is distilled, and the crude product is diluted in xylene and washed with dilute Na₂CO₃. Before the solvent is evaporated, the mixture can be treated with bleaching earths or activated carbon to eliminate the coloured by-products.

### Example 3: Synthesis of N,N'-Bis(nonanoylamido)ethylenediamine

The same process as used for N,N'-Bis(3-aminopropyl)ethylenediamine reported in example 1 is used, starting with 54.3 g of pelargonic acid (96%, 0.33 mol) and 10.0 g of ethylenediamine (0.17 mol). Once again a pale yellow solid is obtained, which can be used without further purification.

Alternatively, this intermediate can be synthesised from ethylenediamine and nonanoyl chloride in the presence of triethylamine.

### Example 4: Synthesis of tetraacetylated N,N'-Bis(nonanoylamido)ethylenediamine (Ib')

The same process is used as for the synthesis of tetraacetylated N,N'-Bis[(3-nonanoylamido)propyl]ethylenediamine (III). In this case, however, at the end of the synthesis the product precipitates cold in acetic anhydride, and a white solid is obtained.

### Example 5: Preparation and efficacy test of a powdered detergent containing bleaching activators of formula (I) and oxygenating agents such as sodium perborate tetrahydrate.

The washing test was performed with a washing machine simulator called Linitest (LAUNDER-O-METER);
Using the washing machine simulator we washed, in water, pieces of EMPA 211 fabric (percale cotton) and pieces of standard soiled fabric (EMPA 167 and 164) with 3.5 g/l of detergent (detergent formulation 1) containing the compounds of formula (I). The stain-removal power was compared with a wash performed without activators at two different temperatures, 40°C and 60°C.

The stain-removal power was evaluated by establishing the colorimetric coordinates of the fabric before and after washing.

Using these parameters, the movement of the washed stain compared with the initial unwashed stain and the clean fabric can be established:
The final values reported are in ΔE''' [ΔE''' = ΔE'-ΔE"= (E clean fabric - E unwashed stain)-(E clean fabric - E washed stain)]. In this way the movement of the washed stain compared with the initial unwashed stain and the acceptor fabric (Empa 211) can be established.

The higher the ΔE''', the greater the stain-removal power of the product.

The colorimetric coordinates were read instrumentally using a Datacolor Instrument 650 colorimeter (illuminant observer D65 10 Deg) 0% UV.

The washing test was performed under the following conditions

### LAUNDER-O-METER

Temperature: 40°C, 60°C
Washing time: 30 minutes
Stains washed: EMPA 167, cotton fabric with standard tea stain
   EMPA 164, cotton fabric with standard grass stain
Hardness of water: 20°F
Detergent concentration: 3.5 g/l

| Detergent formulation 1 | % w/w |
|---|---|
| Anionic surfactants | 12 |
| Non-ionic surfactants | 7 |
| Sodium carbonate | 10 |
| Zeolite A | 34 |
| Sodium polyacrylate | 2.1 |
| Antifoaming agent | 3.6 |
| Corrosion inhibitor | 2.4 |
| Sodium sulphate | 13 |

| Composition of stain-removal system: | |
|---|---|
| polyamine of formula (I) | 8.3 |
| sodium perborate tetrahydrate | 8 |

### Results

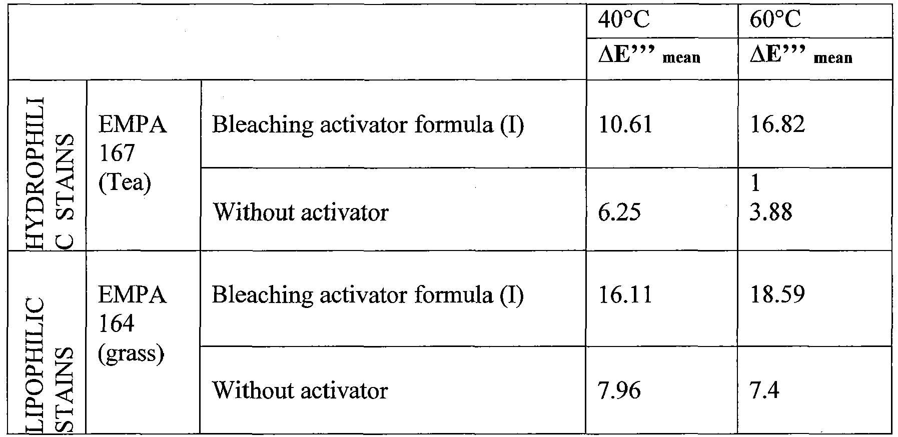

The colour change (ΔE''') obtained with the detergent of formula I containing bleaching activators of formula (I) was greater in the presence of hydrophilic and lipophilic stains than when the detergent without activator was used. In the presence of hydrogen peroxide generators, the compounds of formula (I) therefore act indiscriminately against both lipophilic and hydrophilic stains.

### Example 6: Preparation and efficacy test of a powdered detergent containing bleaching activators of formula (I) and oxygenating agents such as sodium perborate tetrahydrate.

The washing test was again performed with a washing machine simulator called Linitest (LAUNDER-O-METER).

Using the washing machine simulator we washed, in water, pieces of EMPA 211 fabric (percale cotton) and pieces of standard soiled fabric (EMPA 167 and 164) with 3.5 g/l of detergent (detergent formulation 2) containing the compounds of formula (I), adsorbed on Zeolite A. A soak test in water was also performed for 16 consecutive hours, without the use of a washing machine simulator, at the same concentrations as reported above. The stain-removal power was compared with a wash performed without activators at two different temperatures, 40°C and 60°C, with a 16-hour soak. The stain-removal power was again evaluated by establishing the colorimetric coordinates of the fabric before and after washing.

The washing test was performed under the following conditions:

### LAUNDER-O-METER

| | |
|---|---|
| Temperature: | 40°C, 60°C, 16 h soak |
| Washing time: | 30 minutes |
| Stains washed: | EMPA 167, cotton fabric with standard tea stain |
| | EMPA 164, cotton fabric with standard grass stain |
| Hardness of water: | 20°F |
| Detergent concentration: | 3.5 g/l |

| Detergent formulation 2 | % w/w |
|---|---|
| Anionic surfactants | 10 |
| Non-ionic surfactants | 5 |
| Sodium carbonate | 10 |
| Zeolite A | 12.4 |
| Propylene glycol | 1.6 |
| Sodium polyacrylate | 2.1 |
| Antifoaming agent | 3.6 |
| Corrosion inhibitor | 2.4 |
| Sodium sulphate | 13 |

| Composition of stain-removal system: | |
|---|---|
| polyamine of formula (I) | 8.3 |
| sodium perborate tetrahydrate | 8 |
| Zeolite A | 23.6 |

The values shown are in ΔE''' [ΔE''' = ΔE'-ΔE"= (E clean fabric - E unwashed stain)-(E clean fabric - E washed stain)]. In this way the movement of the washed stain compared with the initial unwashed stain and the acceptor fabric (Empa 211) can be established.

### Results

| Stain | Conditions | Bleaching activator formula (I) | without activator |
|---|---|---|---|
| | | Δ**E''' _{Mean}** | **ΔE''' ₘₑₐₙ** |
| EMPA 164 -grass- | 40°C | 10.04 | 6.5 |
| | 60°C | 15.64 | 13.8 |
| | 16h soak | 18.3 | 15.6 |
| EMPA 167 -tea- | 40°C | 15.85 | 7.95 |
| | 60°C | 16.22 | 7.4 |
| | 16h soak | 15.4 | 13.8 |

The colour change (ΔE''') obtained with the detergent of formula II containing bleaching activators of formula (I) was greater in the presence of hydrophilic and lipophilic stains than when the detergent without activator was used. In the presence of hydrogen peroxide generators, the compounds of formula (I) therefore act indiscriminately against both lipophilic and hydrophilic stains.

## Claims

1. A compound of formula I 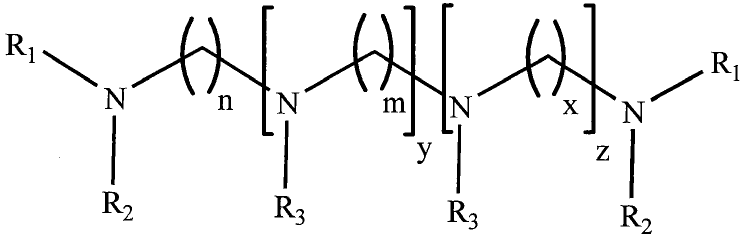
wherein R₁ is a straight or branched, saturated or unsaturated, acyl group having 2 to 5 carbon atoms;
R₂ is a straight or branched, saturated or unsaturated acyl group having 6 to 18 carbon atoms;
R₃ is a straight or branched, saturated or unsaturated acyl or alkyl group, containing 2 to 18 carbon atoms;
y is an integer from 0 to 3, m, n and x are independently an integer from 2 to 3; z is zero or 1.

2. A compound according to claim 1 wherein R₃ is the same as R₁ or R₂.

3. A compound according to claim 1 wherein R₁ is an acetyl group.

4. A compound according to claim 1 wherein R₂ is a straight saturated acyl group having 8 to 12 carbon atoms, preferably nonanoyl.

5. A compound according to claim 1 wherein z is 1 of formula (Ia) 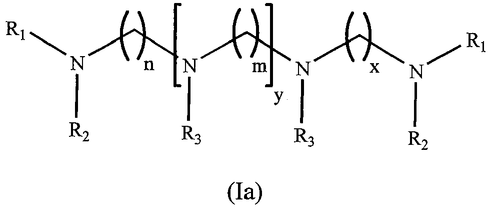 wherein R₁, R₂, R₃, n, m, x and y are as defined in claim 1.

6. A compound according to claim 1 wherein y and z are 0, of formula (Ib). 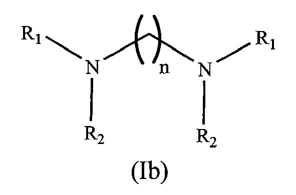 wherein R₁, R₂ and n are as defined in claim 1.

7. Bleaching compositions which comprise a compound of claims 1-6 in a mixture with conventional ingredients.

8. A bleaching composition according to claim 7 containing a peroxygenated compound capable of producing hydrogen peroxide in aqueous medium.

9. A composition according to claim 8 wherein the ratio of peroxygenated compound to compound of formula I ranges from 50:1 to 1:50.

10. A composition according to claim 8 or 9 wherein the peroxygenated compound is selected from sodium perborate mono- or tetra- hydrate, sodium carbonate peroxyhydrate, sodium percarbonate, sodium pyrophosphate peroxyhydrate, urea peroxyhydrate, sodium peroxide, hydrogen peroxide or mixtures thereof.

11. A bleaching composition according to one or more of claims 1 to 10 containing:
(a) 1% to 70% by weight of one or more peroxygenated compounds able to produce hydrogen peroxide in aqueous solution;
(b) 0.1% to 60% by weight of one or more surfactants;
(c) 0.1% to 60% by weight of a compound of claims 1-6.

12. A composition according to claim 11 further comprising sequestering detergents.

13. A composition according to claim 12 wherein the sequestering detergent is an aluminosilicate or a zeolite, preferably Zeolite A.

14. A composition according to claim 13 wherein the compounds of claims 1-6 are adsorbed on the aluminosilicate or zeolite.

15. A composition according to claims 7-14 containing 0.001 to 30% by weight of bleach catalysts.

## Patentansprüche

1. Verbindung der Formel I 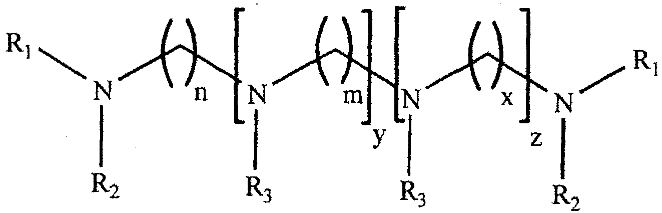
worin R₁ eine geradkettige oder verzweigte, gesättigte oder ungesättigte Acylgruppe mit 2 bis 5 Kohlenstoffatomen ist;
R₂ eine geradkettige oder verzweigte, gesättigte oder ungesättigte Acylgruppe mit 6 bis 18 Kohlenstoffatomen ist;
R₃ eine geradkettige oder verzweigte, gesättigte oder ungesättigte Acyl- oder Alkylgruppe, die 2 bis 18 Kohlenstoffatome enthält, ist;
y eine ganze Zahl von 0 bis 3 ist, m, n und x unabhängig eine ganze Zahl von 2 bis 3 sind; z 0 oder 1 ist.

2. Verbindung gemäß Anspruch 1, wobei R₃ das gleiche wie R₁ oder R₂ ist.

3. Verbindung gemäß Anspruch 1, wobei R₁ eine Acetylgruppe ist.

4. Verbindung gemäß Anspruch 1, wobei R₂ eine geradkettige gesättigte Acylgruppe mit 8 bis 12 Kohlenstoffatomen, vorzugsweise Nonanoyl, ist.

5. Verbindung gemäß Anspruch 1, worin z 1 ist, der Formel (Ia), 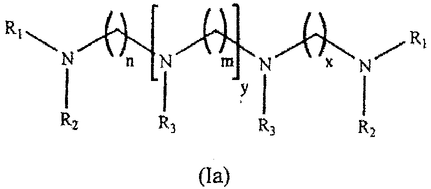 worin R₁, R₂, R₃, n, m, x und y wie in Anspruch 1 definiert sind.

6. Verbindung gemäß Anspruch 1, worin y und z 0 sind, der Formel (Ib), 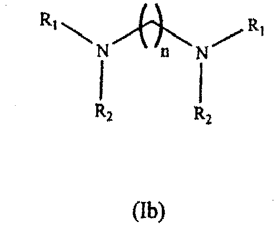 worin R₁, R₂ und n wie in Anspruch 1 definiert sind.

7. Bleichmittelzusammensetzungen, die eine Verbindung der Ansprüche 1 bis 6 in einem Gemisch mit konventionellen Inhaltsstoffen umfassen.

8. Bleichmittelzusammensetzung gemäß Anspruch 7, die eine peroxygenierte Verbindung, die in der Lage ist, in einem wässrigen Medium Wasserstoffperoxid zu erzeugen, enthält.

9. Zusammensetzung gemäß Anspruch 8, wobei das Verhältnis an peroxygenierter Verbindung zu Verbindung der Formel I von 50:1 bis 1:50 reicht.

10. Zusammensetzung gemäß Anspruch 8 oder 9, wobei die peroxygenierte Verbindung ausgewählt ist aus Natriumperboratmono- oder -tetrahydrat, Natriumcarbonatperoxyhydrat, Natriumpercarbonat, Natriumpyrophosphatperoxyhydrat, Harnstoffperoxyhydrat, Natriumperoxid, Wasserstoffperoxid oder Mischungen davon.

11. Bleichmittelzusammensetzung gemäß einem oder mehreren der Ansprüche 1 bis 10, enthaltend:
(a) 1 Gew.-% bis 70 Gew.-% einer peroxygenierten Verbindung oder mehrerer peroxygenierter Verbindungen, die in der Lage ist/sind, in wässriger Lösung Wasserstoffperoxid zu erzeugen;
(b) 0,1 Gew.-% bis 60 Gew.-% eines Tensids oder mehrerer Tenside;
(c) 0,1 Gew.-% bis 60 Gew.-% einer Verbindung der Ansprüche 1 bis 6.

12. Zusammensetzung gemäß Anspruch 11, die weiterhin Sequestrieren der Detergenzien umfasst.

13. Zusammensetzung gemäß Anspruch 12, wobei das sequestrierende Detergens ein Aluminosilicat oder ein Zeolith, vorzugsweise Zeolith A, ist.

14. Zusammensetzung gemäß Anspruch 13, wobei die Verbindungen der Ansprüche 1 bis 6 an dem Aluminosilicat oder Zeolithen adsorbiert sind.

15. Zusammensetzung gemäß den Ansprüchen 7 bis 14, die 0,001 Gew.-% bis 30 Gew.-% an Bleichkatalysatoren enthält.

## Revendications

1. Composé de formule I 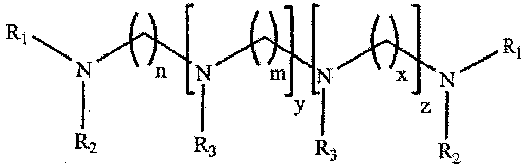
dans laquelle R₁ est un groupe acyle linéaire ou ramifié, saturé ou insaturé, ayant de 2 à 5 atomes de carbone ;
R₂ est un groupe acyle linéaire ou ramifié, saturé ou insaturé ayant de 6 à 18 atomes de carbone ;
R₃ est un groupe acyle ou alkyle linéaire ou ramifié, saturé ou insaturé, contenant de 2 à 18 atomes de carbone ;
y est un nombre entier d'une valeur de 0 à 3, m, n et x sont indépendamment un nombre entier d'une valeur de 2 à 3 ; z a la valeur de zéro ou 1.

2. Composé selon la revendication 1 dans lequel R₃ est identique à R₁ ou R₂.

3. Composé selon la revendication 1 dans lequel R₁ est un groupe acétyle.

4. Composé selon la revendication 1 dans lequel R₂ est un groupe acyle linéaire saturé ayant de 8 à 12 atomes de carbone, préférablement un groupe nonanoyle.

5. Composé selon la revendication 1 dans lequel z est 1 de formule (la) 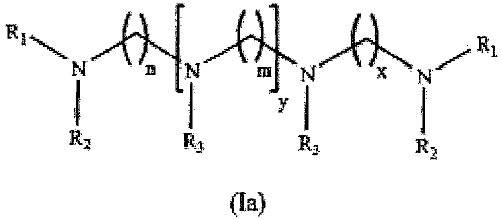 où R₁, R₂, R₃, n, m, x et y sont tels que définis selon la revendication 1.

6. Composé selon la revendication 1 dans lequel y et z sont 0, de formule (Ib). 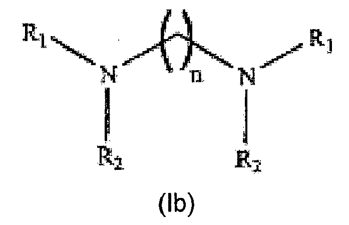 où R₁, R₂ et n sont tels que définis selon la revendication 1.

7. Compositions de blanchissage qui comprennent un composé selon les revendications 1 à 6 dans un mélange avec des ingrédients classiques.

8. Composition de blanchissage selon la revendication 7 contenant un composé peroxygéné capable de produire du peroxyde d'hydrogène en milieu aqueux.

9. Composition selon la revendication 8 dans laquelle le rapport du composé peroxygéné au composé de formule I s'étend de 50:1 à 1:50.

10. Composition selon la revendication 8 ou 9 dans laquelle le composé peroxygéné est sélectionné parmi le perborate de sodium mono- ou tétrahydraté, le carbonate de sodium peroxyhydraté, le percarbonate de sodium, le pyrophosphate de sodium peroxyhydraté, le peroxyhydrate d'urée, le peroxyde de sodium, le peroxyde d'hydrogène ou leurs mélanges.

11. Composition de blanchissage selon l'une ou plusieurs des revendications 1 à 10 contenant :
(a) de 1 % à 70 % en poids d'un ou plusieurs composés peroxygénés capables de produire du peroxyde d'hydrogène en solution aqueuse ;
(b) de 0,1 % à 60 % en poids d'un ou plusieurs tensioactifs ;
(c) de 0,1 % à 60 % en poids d'un composé selon les revendications 1 à 6.

12. Composition selon la revendication 11 comprenant en outre des détergents de séquestration.

13. Composition selon la revendication 12 dans laquelle le détergent de séquestration est un aluminosilicate ou une zéolite, préférablement de la zéolite A.

14. Composition selon la revendication 13 dans laquelle les composés selon les revendications 1 à 6 sont adsorbés sur de l'aluminosilicate ou de la zéolite.

15. Composition selon les revendications 7 à 14 contenant de 0,001 à 30 % en poids de catalyseurs de blanchissage.
